# FASCICULE DE BREVET EUROPEEN

(11) **EP 2 282 666 B1**
(45) Date de publication et mention de la délivrance du brevet: **27.04.2016**
(21) Numéro de dépôt: 09738134.7
(22) Date de dépôt: 28.04.2009
(51) Int. Cl.: A61B 5/00

(54) **DISPOSITIF DE MESURE DE PRESSION ET/OU DE TEMPERATURE INTERROGEABLE A DISTANCE IMPLANTABLE EN MILIEU BIOLOGIQUE**
VON FERN ANSPRECHBARE DRUCK- UND/ODER TEMPERATURMESSVORRICHTUNG, DIE IN EINEM BIOLOGISCHEM MEDIUM INSTALLIERT IST
REMOTELY ADDRESSABLE PRESSURE AND/OR TEMPERATURE MEASURING DEVICE INSTALLED IN A BIOLOGICAL MEDIUM

(30) Priorité: 30.04.2008 FR 0802419
(43) Date de publication de la demande: 16.02.2011
(73) Titulaire: Senseor, 06560 Valbonne (FR)
(72) Inventeur: CHOMMELOUX, Luc, F-06110 Le Cannet (FR); MENAGE, Philippe, F-06140 Vence (FR); HEIDER, Gerhard, F-06560 Le Rouret (FR)
(74) Mandataire: Esselin, Sophie
(86) Numéro de dépôt international: PCT/EP2009/055103
(87) Numéro de publication internationale: WO 2009/133090

(56) Documents cités:
- WO-A-00/56210
- WO-A-2004/014456
- US-A- 6 053 873
- US-A1- 2002 151 816

## Description

L'invention se rapporte à un nouveau type de dispositifs à base de capteurs, sans fil et sans batterie, permettant notamment la mesure de la pression artérielle et de la température sanguine, ledit capteur étant implantable directement dans un milieu biologique.

Il s'agit plus précisément de capteur passif interrogeable à distance via une antenne couplée et avantageusement utilisable dans tout type de milieu organique présentant une permittivité diélectrique notablement supérieure à celle du vide. Typiquement sont concernés les milieux biologiques pour lesquels il est particulièrement intéressant de disposer de capteurs permettant des mesures de pression type pression artérielle.

De manière générale, la forte permittivité diélectrique des milieux biologiques permet de concevoir des capteurs passifs notamment à ondes de surface fonctionnant à des longueurs d'onde électromagnétique réduites et autorisant par la même des dimensions d'antennes également réduites.

Il a déjà été proposé des solutions notamment décrites dans la demande de brevet BF Goodrich WO 00/56210, dans lesquelles un capteur passif de pression à ondes acoustiques de surface dénommé couramment « SAW » solidaire d'un stent permet en particulier de mesurer la pression sanguine chez l'animal ou l'homme.

Dans le cas considéré, le mode d'interrogation entre l'émetteur/récepteur situé à l'extérieur de l'être vivant et le capteur implanté s'effectue par couplage inductif. Ce mode d'interrogation couramment utilisé dans le cadre des capteurs sur silicium passif utilise des fréquences pouvant aller jusqu'à la bande ISM 13,56 MHz. Au-delà de ces fréquences les pertes : pertes magnétiques, pertes par courant de Foucault (qui augmentent avec le carré de la fréquence), pertes par effet joules deviennent très importantes et limitent les distances d'interrogation à des valeurs qui ne présentent plus d'intérêt vis-à-vis des applications visées.

Il n'est cependant pas possible d'utiliser la bande ISM à 13,56 MHz pour interroger ce type de dispositif. En effet, la taille d'un résonateur SAW à cette fréquence devrait être de l'ordre de 50 mm sur 10 mm ce qui rend impossible une quelconque implantation dans l'artère d'un être humain en particulier.

Afin de satisfaire le compromis taille de capteur (possibilité d'implantation) - distance d'interrogation, la présente demande propose un dispositif de mesure de pression et/ou de température utilisant une méthode 5 d'interrogation alternative basée sur le rayonnement électromagnétique d'une antenne fonctionnant à des fréquences supérieures par rapport à la bande ISM à 13,56 MHz. A titre d'exemple il peut s'agir d'un système fonctionnant dans la bande ISM à 434 MHz. Dans ces conditions la taille d'un résonateur peut être typiquement de l'ordre de 2,5 mm par 0,5 mm ce qui rend tout à fait possible l'implantation in vivo d'un tel dispositif.

WO 2004/014456 A2 décrit un dispositif selon le préambule de la revendication 1.

Dans ce contexte, la présente invention propose un nouveau type de dispositif implantable comportant un capteur passif à base de SAW et capable d'être introduit notamment dans une artère et ainsi susceptible de fournir des informations de pression artérielle et/ou de température sanguine.

Plus précisément la présente invention a pour objet un dispositif de mesure de pression et de température interrogeable à distance selon la révendication 1.

Selon une variante de l'invention, que le système d'interrogation fonctionne dans la bande ISM à 434 MHz.

Selon une variante de l'invention, la structure tubulaire comprend une structure métallique en treillis expansible.

Selon une variante de l'invention, le capteur est situé sur une paroi de la structure tubulaire.

Selon une variante de l'invention, le matériau biocompatible est une résine ou un élastomère.

Selon une variante de l'invention, la structure tubulaire comporte une partie rigide non expansible dans laquelle est intégré le capteur.

Selon une variante de l'invention, l'élément d'antenne appartient à une structure tubulaire métallique.

La compacité de la solution SAW et notamment à 434 MHz permet également de proposer un capteur qui permet de mesurer la pression et la température tout en restant compatible des contraintes d'encombrement en considérant une structure utilisant trois résonateurs par exemple. La mesure de la pression et de la température localisée au même point permet effectivement d'améliorer significativement la précision sur la mesure de ces paramètres

Selon une variante de l'invention, chaque résonateur est couplé à une antenne intégrée.

Selon une variante de l'invention, le capteur est un capteur de pression et de température, comportant un empilement de plusieurs substrats comportant chacun un résonateur et une antenne intégrée, réalisé par l'intermédiaire de murs périphériques.

Selon une variante de l'invention, les murs périphériques sont de type pâte de verre.

Selon une variante de l'invention, le résonateur sensible à la pression est en position tête-bêche par rapport au résonateur de référence.

L'invention a aussi pour objet l'utilisation d'un dispositif selon l'invention pour mesurer une pression artérielle et une température sanguine caractérisée en ce que la structure expansible est placée sur un ballon situé à l'extrémité d'un cathéter destiné à être introduit dans une artère.

L'invention a encore pour objet un procédé de fabrication d'un dispositif selon l'invention comprenant les étapes suivantes :
- la réalisation d'une ouverture sur une partie périphérique d'une structure tubulaire expansible;
- la mise en place de ladite structure tubulaire expansible sur un mandrin présentant une rainure;
- le positionnement du capteur dans l'ouverture de la structure tubulaire positionnée sur le mandrin et en regard de ladite rainure ;
- le dépôt d'une résine d'enrobage biocompatible permettant d'encapsuler ledit capteur ;
- le retrait de la structure tubulaire dudit mandrin.

L'invention sera mieux comprise et d'autres avantages apparaîtront à la lecture de la description qui va suivre donnée à titre non limitatif et grâce aux figures annexées parmi lesquelles :
- la figure 1 schématise le fonctionnement du dispositif de l'invention ;
- la figure 2 illustre une vue schématique d'un capteur à ondes de surface équipé de brins d'antenne selon l'art connu ;
- la figure 3 est une vue schématique en coupe d'un capteur à ondes de surface packagé ;
- la figure 4 est une vue schématique en coupe d'un système intégrant un capteur de pression avec substrat piézoélectrique aminci ;
- la figure 5 est une vue schématique en coupe d'un système intégrant deux capteurs à ondes de surface ;
- la figure 6 une vue schématique en coupe d'un dispositif implantable selon l'invention, comprenant trois capteurs à ondes de surface montés sur un support biocompatible ;
- les figures 7a à 7f illustrent les différentes étapes d'un exemple de procédé de fabrication d'un dispositif de l'invention ;
- les figures 8, 9 et 10 sont des vues schématiques en coupe de différentes étapes de mise en place d'un dispositif de l'invention au sein d'une artère ;
- la figure 11 illustre une vue schématique en coupe d'un dispositif selon l'invention implanté dans une artère.

L'invention propose une solution innovante de capteurs, sans fil et sans batterie, permettant notamment la mesure de la pression artérielle et de la température sanguine implantable directement dans un milieu biologique, typiquement dans une artère humaine.

Le principe de capteur passifs à ondes de surface utilisé dans la présente invention est décrit ci-après. Il peut selon l'art connu s'agir notamment de capteur de température et/ou de pression.

De manière générale, un système complet se compose d'une unité d'interrogation (constituée elle-même d'une partie émetteur et d'une partie récepteur i .e . E/R) et d'un capteur de température et/ou de pression à ondes acoustiques de surface SAW. Le dispositif SAW est de type résonateur ce qui permet d'accéder à des structures de tailles réduites. Le système d'interrogation ainsi que le capteur SAW sont munis respectivement d'antennes, adaptées à la bande de fréquence de travail (bandes ISM 433 MHz, 868 MHz, 2.45 GHz,...) ou à toute autre bande de fréquence libre d'utilisation, ce qui permet d'effectuer une interrogation sans fil du capteur.

Le mode d'interrogation est le suivant : l'émetteur du système d'interrogation envoie un signal d'interrogation (impulsion temporelle d'une porteuse dans la bande ISM, plage temporelle d'émission) vers l'antenne associée au résonateur SAW. Par effet de couplage piézoélectrique, l'onde électromagnétique incidente est transformée en une onde acoustique se propageant à la surface du substrat.

Si le signal d'émission présente une fréquence de résonance suffisamment proche de la fréquence propre du résonateur SAW, ce dernier entre en résonance en passant par une période de charge. Il s'établit alors un régime permanent d'oscillations à la fréquence de résonance propre du dispositif SAW. Cette fréquence de résonance est proportionnelle à la vitesse de l'onde de surface dans la cavité résonante qui dépend elle-même de la température et des contraintes vues par le résonateur.

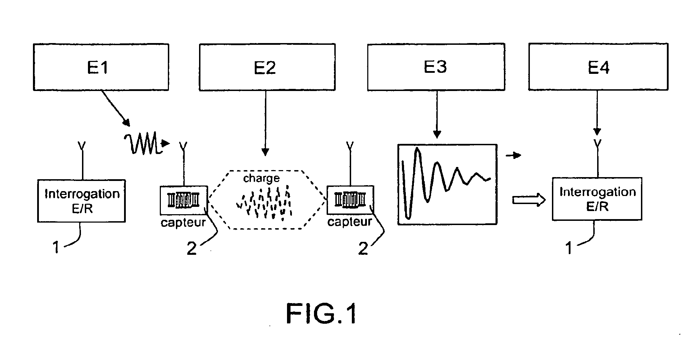

Le fonctionnement du dispositif de l'invention est le suivant : le système complet illustré en figure 1, se compose d'une unité d'interrogation 1 (constituée elle-même d'une partie émetteur et d'une partie récepteur i .e . E/R) et du capteur SAW 2 constitué d'un ou plusieurs résonateurs fonctionnant à des fréquences différentes. Le système d'interrogation ainsi que le capteur SAW sont munis d'une antenne adaptée à la bande de fréquence de travail ce qui permet d'effectuer une interrogation sans fil du capteur.

Le mode d'interrogation est le suivant : durant une première étape E1, l'émetteur du système d'interrogation 1 envoie un signal d'interrogation (impulsion temporelle d'une porteuse dans la bande de fréquence adaptée au capteur) vers le capteur SAW 2. Cette impulsion présente une largeur spectrale inférieure à la bande passante du résonateur.

Durant une étape E2, si le signal d'émission présente une fréquence de résonance suffisamment proche de la fréquence propre du résonateur SAW, ce dernier entre en résonance en passant par une période de charge, pour atteindre un régime permanent d'oscillation. Cette fréquence de résonance est en particulier fonction de la vitesse de l'onde de surface dans la cavité résonante qui dépend des conditions de pression et de température vues par le résonateur.

Durant une étape E3, on procède à la lecture de la réponse du résonateur avant sa décharge.

Le capteur rayonne à son tour (via l'antenne qui lui est connectée) un signal à sa fréquence de résonance qui porte l'information de pression et de température, correspondant à l'étape E4.

Le récepteur du système d'interrogation détecte en dehors de la plage temporelle d'émission tout ou partie du signal SAW (oscillation amortie) et en extrait l'information de pression et/ ou de température recherchée via un traitement du signal adapté.

Afin de détecter les différentes fréquences de résonance du capteur, un balayage complet de la bande ISM est effectué avec un certain pas fréquentiel qui est aussi inférieur à la bande passante du résonateur.

Ce mode de communication entre l'interrogateur et le capteur permet d'obtenir des distances d'interrogation supérieure au mètre tout en respectant les exigences radio de la norme ISM.

Nous allons décrire ci-après plus en détails un capteur utilisé dans un dispositif de l'invention comportant plusieurs résonateurs à ondes de surface, permettant la mesure de la pression et de la température in situ.

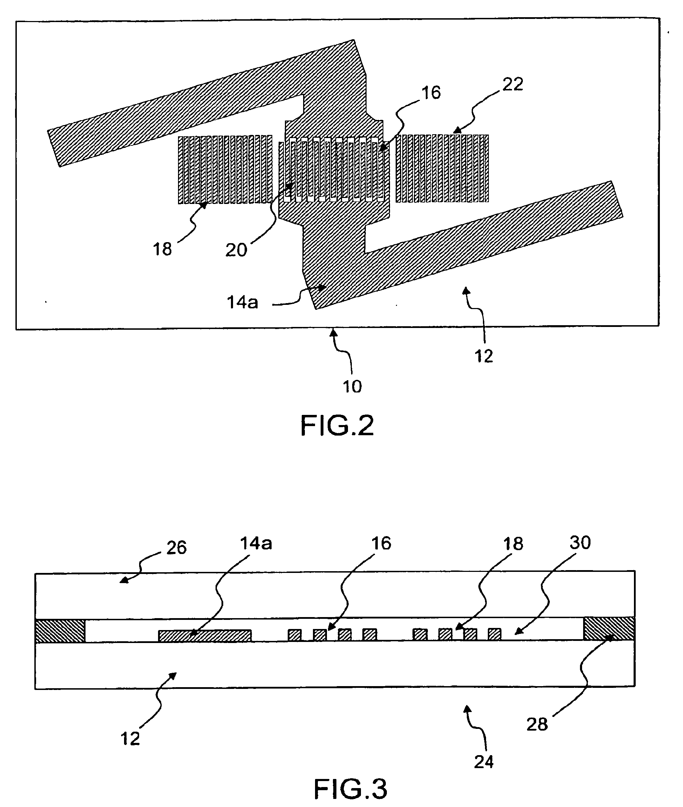

La figure 2 illustre une vue schématique de dessus d'un capteur à ondes de surface 10 constitué d'un substrat piézoélectrique 12, d'une structure résonante, appelée résonateur et de deux brins d'antenne d'émission 14a. Le résonateur est composé d'un transducteur 16 et de deux ensembles de réflecteurs 18. Le substrat piézoélectrique 12 peut être du Quartz, du Niobate de Lithium (LiNbO₃), du Tantalate de Lithium (LiTaO₃) ou tout autre matériau piézoélectrique.

La partie transducteur 16 du résonateur est constituée d'une alternance d'électrodes métalliques 20, appelé transducteur interdigité (IDT pour InterDigital Transducer), alternativement connectées aux deux brins de l'antenne 14a. Les deux ensembles de réflecteurs 18 sont constitués d'une alternance d'électrodes métalliques 22.

La largeur des électrodes métalliques 20 du transducteur 16, leur espacement, la largeur des lignes métalliques 22 des ensembles de réflecteurs 18, leur espacement, leur nombre ainsi que le type de substrat piézoélectrique 12 permettent de définir les caractéristiques du résonateur telles que la fréquence de résonance par exemple. Ainsi, chaque capteur (pression et température) est défini pour résonner à une fréquence particulière. L'application d'une pression et/ou d'une température sur le substrat piézoélectrique 12 engendre une variation de la fréquence de résonance du capteur. La mesure du paramètre physique se fait alors en comparant la fréquence du résonateur de pression/température à la fréquence d'un résonateur appelé référence. Un système de capteurs de température et de pression peut comprendre avantageusement trois résonateurs (pression, température et référence), réalisés sur trois substrats piézoélectriques différents. Il est aussi possible d'avoir deux résonateurs (référence et pression par exemple) sur le même substrat piézoélectrique.

Les différents éléments métalliques que sont les électrodes métalliques 20 du transducteur 16, les lignes métalliques 22 de deux ensembles de réflecteurs 18 et les deux brins d'antenne 14a, peuvent être réalisés par différentes technologies de photolithographie, largement connues dans le domaine des semi conducteurs. Les métaux, ainsi que leurs alliages , utilisables pour la réalisation de ces différents éléments sont, par exemple, l'aluminium (Al), le tungstène (W), le platine (Pt), le cuivre (Cu), le titane (Ti) ou tout autre métal ou alliage compatible des procédés de photolithographie et compatibles des applications de résonateurs à onde de surface.

La réalisation de deux brins d'antennes 14a permet d'obtenir un système de mesure de pression et de température, interrogeable à distance. Il est important de noter que l'antenne peut être directement intégrée avec les résonateurs. Dans certains cas où celle-ci ne peut pas être réalisée sur le substrat piézoélectrique, le support correspondant à la structure tubulaire sur laquelle est fixé le capteur peut également servir de support pour l'antenne, si cette structure est par exemple métallique.

Une variante de l'invention consiste à modifier la structure de la structure tubulaire pour que celle-ci fasse office d'antenne.

La figure 3 illustre une vue schématique en coupe d'un capteur à ondes de surface packagé 24 en vue d'une implantation dans un milieu biologique.

Ce packaging est composé d'un capot 26 et de murs 28, séparant le substrat piézoélectrique 12 du capot 26, formant ainsi une cavité hermétique 30.

Le capot 26 est constitué d'un matériau aux caractéristiques mécaniques et diélectriques identiques à celles du substrat piézoélectrique. Dans la plupart des cas, le matériau du capot est le même que le substrat piézoélectrique.

Les murs 28 sont réalisés par différentes techniques telles que le frittage de pâte de scellement, préalablement déposée par sérigraphie par exemple. Une fois le scellement entre le capot et le substrat piézoélectrique réalisé, les murs 28 permettent d'assurer une cavité 30 entre les deux substrats, cavité nécessaire à la propagation des ondes de surface. En fonction des applications envisagées, il est possible de faire varier la pression à l'intérieur de la cavité. Cette pression sert alors de référence par rapport à la pression à mesurer.

Ce procédé de fabrication, réalisé de manière collective, est très largement répandu dans l'industrie du semi conducteur et est couramment appelé MEMS (pour Micro-Electro-Mechanical Systems).

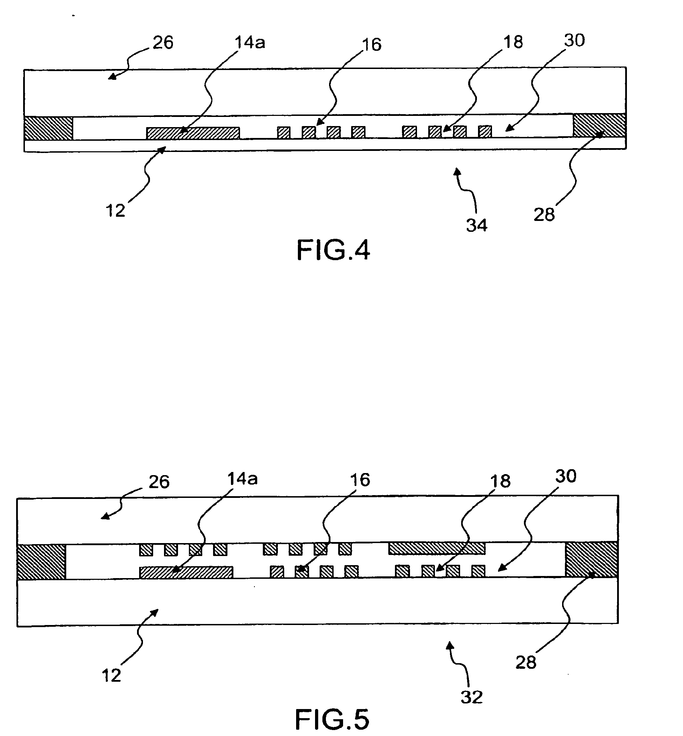

La figure 4 est une vue schématique en coupe d'un système intégrant un capteur de pression avec substrat piézoélectrique aminci 34.

L'étape d'amincissement du substrat piézoélectrique 12 est principalement réalisée sur le capteur de pression en vue d'obtenir la sensibilité voulue aux variations de pression. L'opération d'amincissement peut être réalisée de différentes manières, collectives ou unitaires, telles que le polissage mécanique, le rodage (dénommé couramment « grinding »), la gravure (dénommé couramment « etching ») par exemple.

Cette option peut être aussi envisagée pour des raisons de réduction de taille en vue d'une implantation en milieu biologique.

La figure 5 est une vue schématique en coupe d'un système intégrant deux capteurs à ondes de surface 32, positionnés tête-bêche. Cette option peut être envisagée pour des raisons de réduction de taille en vue d'une implantation en milieu biologique.

Les différents capteurs décrits ci-dessus peuvent avantageusement être intégrés dans un dispositif de l'invention, et plus précisément au niveau d'une structure tubulaire métallique de type stent destiné notamment à être introduit au sein d'une artère pour en mesurer la pression artérielle.

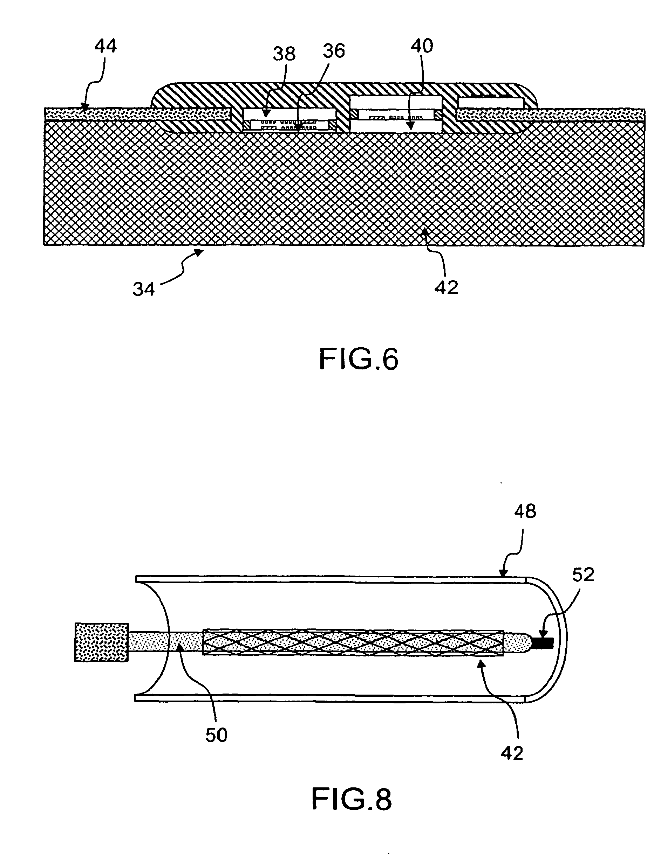

La figure 6 illustre une vue schématique en coupe d'un dispositif implantable selon l'invention, comprenant trois capteurs à ondes de surface montés sur un support biocompatible 34. Les trois capteurs utilisés ici sont un capteur de pression, sur substrat aminci, 36, assemblé avec le capteur de référence 38 et un capteur de température 40.

Ces trois capteurs sont positionnés/intégrés dans un stent métallique 42 dont la particularité est d'avoir une zone rigide non déformable 44. Les capteurs sont englobés dans une résine de protection biocompatible 46, déposée à l'aide d'une seringue par exemple.

Avantageusement, une antenne non intégrée est intégrée au stent 42, par exemple dans la zone rigide du stent 42. Cette antenne peut aussi être englobée dans une résine de protection biocompatible 46. Une alternative de cette variante de l'invention consiste à se servir du stent 42 comme antenne.

Il est rappelé que, de manière générale, un stent est un petit treillis métallique extensible qui est glissé dans une cavité naturelle (artère,...) pour la maintenir ouverte.

Il est essentiellement utilisé dans des artères au cours d'une angioplastie (technique, médico-chirurgicale de modification/augmentation du diamètre d'un vaisseau sanguin couramment réalisée dans le cas de maladies pour lesquelles les diamètres des vaisseaux sanguins sont affectés (occlusion, sténoses,...).

Néanmoins, le stent étant métallique, donc constituant un corps métallique étranger, il peut entraîner l'apparition de caillot par agrégat plaquettaire. Des traitements médicamenteux (antiagrégants plaquettaires) sont donc nécessaires pour empêcher l'apparition de ces problèmes. Afin de réduire le risque d'apparition de caillot, le stent est protégé par une résine biocompatible qui vient englober partiellement ou entièrement le métal.

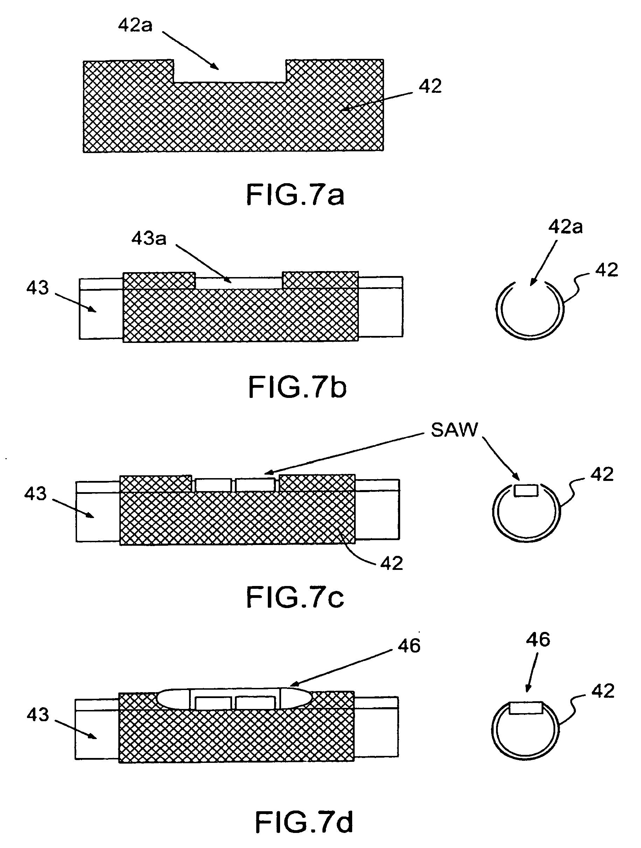

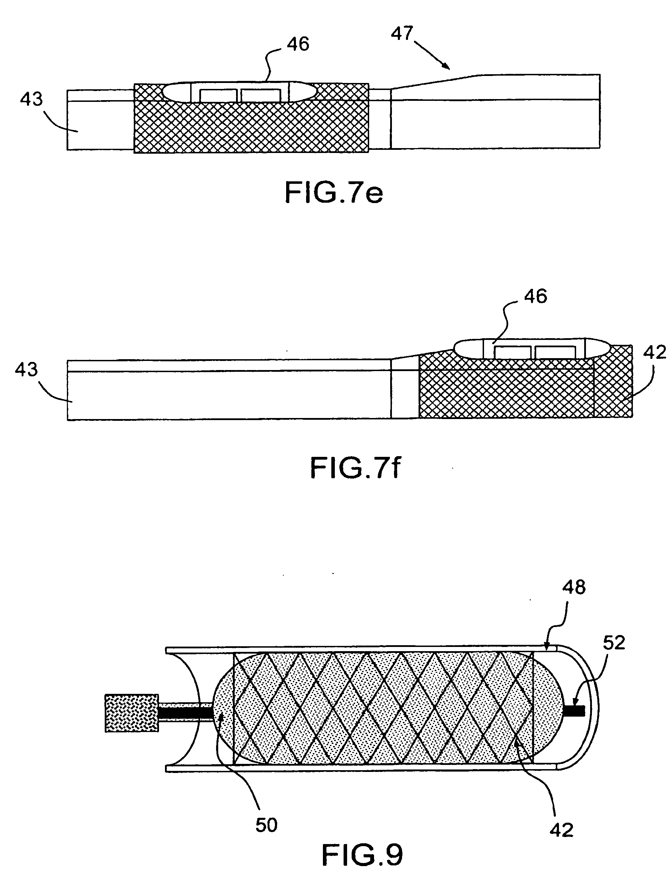

Nous allons décrire ci-après un exemple de procédé de fabrication d'un dispositif selon l'invention destiné à être implanté dans une artère, dont les étapes sont illustrés en figures 7a à 7f.
- on réalise dans un premier temps une ouverture rectangulaire 42a au sein du stent 42, permettant le positionnement du capteur. Cette ouverture peut avoir des dimensions en fonction de l'emplacement dans lequel il sera placé dans le corps humain ;
- on procède alors à la mise en place du stent sur un mandrin 43 spécifiquement usiné pour recevoir les capteurs. Cet usinage se compose principalement d'une rainure 43a ;
- on procède alors à la mise en place du capteur à base de SAW dans la zone ouverte du stent par placement effectué par exemple par procédé de report et placement automatisé ;
- le dépôt d'une résine d'enrobage biocompatible 46 peut être fait à l'aide d'une seringue ou par trempage. La résine est ensuite polymérisée sous l'effet de la température.

Pour la mesure de la pression sanguine, il est important que la résine d'encapsulation ne déborde pas sur la face du capteur qui mesure la pression (soit celle qui est à l'intérieur du stent).

Après polymérisation, il est possible de retirer le stent sans endommager les capteurs en ajoutant au mandrin existant un raccord rainuré de diamètre légèrement supérieur à celui du mandrin. On utilise les caractéristiques élastiques du stent. Il est important de noter que le fond de la rainure du raccord se trouve au même niveau que le fond de la rainure du mandrin.

Une fois le stent retiré du raccord, il est prêt à être utilisé en vue d'une implantation et notamment dans le cadre d'angioplastie.

Les figure 8, figure 9 et figure 10 sont des vues schématiques en coupe des étapes de mise en place d'un stent 42 une fois réalisé en vue des applications précitées.

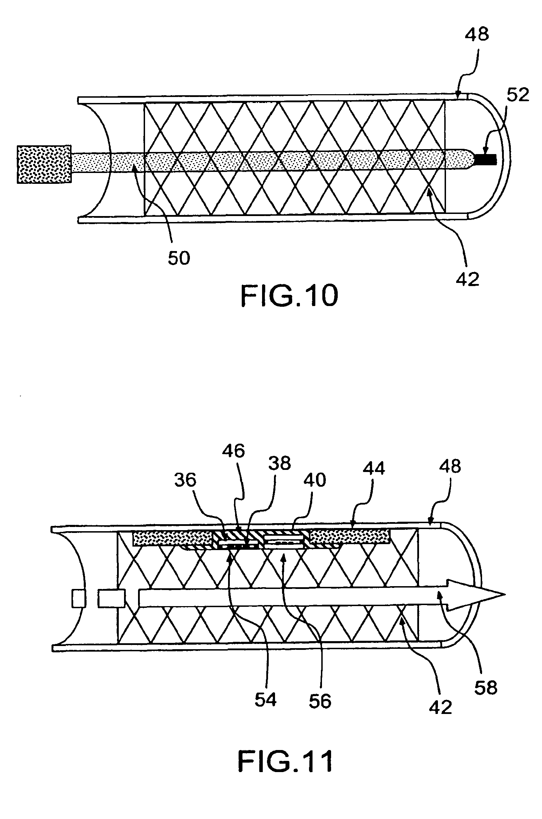

Tout d'abord, le stent est installé sur un ballon dégonflé 50 situé au bout d'un cathéter 52 (figure 8). Le cathéter est ensuite introduit dans l'artère 48. Le ballon 50 est ensuite gonflé (figure 9), ce qui entraîne une expansion du stent 42 qui vient alors se coller à la paroi de l'artère 48. Le ballon 50 est ensuite dégonflé (figure 10) et le cathéter retiré 52.

La figure 11 illustre une vue schématique en coupe du dispositif de l'invention, implanté au niveau d'une artère 48. Les capteurs sont placés contre la paroi de l'artère 48. La surface 54 du capteur de pression 36, surface préalablement amincie, et la surface 56 du capteur de température 40 sont directement en contact avec le flux sanguin circulant dans l'artère 58, permettant la mesure de la pression artérielle et la température sanguine. Le positionnement des capteurs dans une zone rigide du stent (c'est-à-dire non déformable) permet d'éviter les problèmes de désolidarisation de la résine du stent 42 lors de l'opération de gonflage du ballon 50.

Il est à noter que la résine d'encapsulation biocompatible, et hermétique est choisie suffisamment rigide pour éviter que le capteur ne se désolidarise du stent lorsque le ballonnet est gonflé puis dégonflé lors de la pose.

## Revendications

1. Dispositif de mesure de pression et de température interrogeable à distance et comportant au moins :
- un capteur à ondes acoustiques (24) comprenant au moins un résonateur couplé à un premier élément d'antenne, et
- un système d'interrogation comprenant un second élément d'antenne d'émission et de réception;
- ledit dispositif comportant une structure tubulaire expansible (42), ladite structure intégrant un matériau biocompatible (46) et ledit capteur à ondes acoustioues (24) encapsulé dans le matériau biocompatible (46) ;
- le second élément d'antenne fonctionnant à des fréquences supérieures à plusieurs dizaines de MégaHertz.
**caractérisé en ce que** :
- le capteur est un capteur de pression et de température, comportant un premier résonateur de référence (38), un second résonateur pour mesurer la température (40) et un troisième résonateur sensible à la pression (36).

2. Dispositif de mesure de pression et de température, interrogeable à distance selon la revendication 1, **caractérisé en ce que** la structure tubulaire comprend une structure métallique en treillis expansible.

3. Dispositif de mesure de pression et de température, interrogeable à distance selon l'une des revendications 1 ou 2, **caractérisé en ce que** le capteur est situé sur une paroi interne de la structure tubulaire.

4. Dispositif de mesure de pression et de température, interrogeable à distance selon l'une des revendications 1 à 3, **caractérisé en ce que** le matériau biocompatible est une résine.

5. Dispositif de mesure de pression et de température, interrogeable à distance selon l'une des revendications 1 à 4, **caractérisé en ce que** la structure tubulaire comporte une partie rigide non expansible dans laquelle est intégré le capteur.

6. Dispositif de mesure de pression et de température, interrogeable à distance selon l'une des revendications 1 à 5, **caractérisé en ce que** le capteur est un capteur de pression et de température, comportant un empilement de plusieurs substrats comportant chacun un résonateur et une antenne intégrée, réalisé par l'intermédiaire de murs périphériques.

7. Dispositif de mesure de pression et de température, interrogeable à distance selon la revendication 6, **caractérisé en ce que** les murs périphériques sont de type pâte de verre (pv).

8. Dispositif de mesure de pression et de température, interrogeable à distance selon l'une des revendications 6 ou 7, **caractérisé en ce que** le résonateur sensible à la pression est en position supérieure dans l'empilement et en position tête-bêche par rapport au résonateur de référence.

9. Dispositif de mesure de pression et de température, interrogeable à distance selon l'une des revendications précédentes **caractérisé en ce que** le système d'interrogation fonctionne dans la bande ISM à 434 MHz.

10. Procédé de fabrication d'un dispositif de mesure selon l'une des revendications 1 à 9, **caractérisé en ce qu'**il comprend les étapes suivantes :
- la réalisation d'une ouverture sur une partie périphérique d'une structure tubulaire expansible;
- la mise en place de ladite structure tubulaire expansible sur un mandrin présentant une rainure;
- le positionnement du capteur dans l'ouverture de la structure tubulaire positionnée sur le mandrin et en regard de ladite rainure, le capteur comportant un premier résonateur de référence, un second résonateur pour mesurer la température et un troisième résonateur sensible à la pression ;
- le dépôt d'une résine d'enrobage biocompatible permettant d'encapsuler ledit capteur ;
- le retrait de la structure tubulaire dudit mandrin.

## Patentansprüche

1. Fernabfragbares Gerät zum Messen von Druck und Temperatur, das wenigstens Folgendes umfasst:
- einen Akustikwellensensor (24), der wenigstens einen Resonator umfasst, der mit einem ersten Antennenelement gekoppelt ist; und
- ein Abfragesystem, das ein zweites Sende- und Empfangsantennenelement umfasst;
- wobei das Gerät eine expandierfähige tubuläre Struktur (42) umfasst, wobei in der Struktur ein biokompatibles Material (46) integriert und der Akustikwellensensor (24) in dem biokompatiblen Material (46) verkapselt ist;
- wobei das zweite Antennenelement auf Frequenzen über mehreren Dutzend Megahertz arbeitet,
**dadurch gekennzeichnet, dass** der Sensor ein Druck- und Temperatursensor ist, der einen ersten Referenzresonator (38), einen zweiten Resonator (40) zum Messen der Temperatur und einen dritten druckempfindlichen Resonator (36) umfasst.

2. Fernabfragbares Druck- und Temperatmmessgerät nach Anspruch 1, **dadurch gekennzeichnet, dass** die tubuläre Struktur eine expandierfähige metallische Maschengitterstruktur umfasst.

3. Fernabfragbares Druck- und Temperaturmessgerät nach Anspruch 1 oder 2, **dadurch gekennzeichnet, dass** sich der Sensor an einer Innenwand der tubulären Struktur befindet.

4. Fernabfragbares Druck- und Temperaturmessgerät nach einem der Ansprüche 1 bis 3, **dadurch gekennzeichnet, dass** das biokompatible Material ein Harz ist.

5. Fernabfragbares Druck- und Temperaturmessgerät nach einem der Ansprüche 1 bis 4, **dadurch gekennzeichnet, dass** die tubuläre Struktur ein nicht expandierfähiges starres Teil umfasst, in das der Sensor integriert ist.

6. Fernabfragbares Druck- und Temperaturmessgerät nach einem der Ansprüche 1 bis 5, **dadurch gekennzeichnet, dass** der Sensor ein Druck- und Temperatursensor ist, der einen Stapel von mehreren Substraten umfasst, die jeweils einen Resonator und eine integrierte Antenne umfassen, produziert mittels peripherer Wände.

7. Fernabfragbares Druck- und Temperatmmessgerät nach Anspruch 6, **dadurch gekennzeichnet, dass** die peripheren Wände vom Glaspastentyp (pv) sind.

8. Fernabfragbares Druck- und Temperaturmessgerät nach Anspruch 6 oder 7, **dadurch gekennzeichnet, dass** sich der druckempfindliche Resonator in einer oberen Position in dem Stapel und Kopf an Fuß relativ zu dem Referenzresonator befindet.

9. Fernabfragbares Druck- und Temperatmmessgerät nach einem der vorherigen Ansprüche, **dadurch gekennzeichnet, dass** das Abfragesystem im ISM-Band bei 434 MHz arbeitet.

10. Verfahren zur Herstellung eines Messgeräts nach einem der Ansprüche 1 bis 9, **dadurch gekennzeichnet, dass** es die folgenden Schritte beinhaltet:
- Erzeugen einer Öffnung an einem peripheren Teil einer expandierfähigen tubulären Struktur;
- Platzieren der expandierfähigen tubulären Struktur auf einem Dorn mit einer Nut;
- Positionieren des Sensors in der Öffnung der tubulären Struktur, die auf dem Dorn positioniert und der Nut zugewandt ist, wobei der Sensor einen ersten Referenzresonator, einen zweiten Resonator zum Messen der Temperatur und einen dritten druckempfindlichen Resonator umfasst;
- Absetzen einer biokompatiblen Harzbeschichtung, die eine Verkapselung des Sensors zulässt;
- Abnehmen der tubulären Struktur von dem Dorn.

## Claims

1. A remotely interrogatable pressure and temperature measuring device comprising at least:
- one acoustic wave sensor (24) comprising at least one resonator coupled to a first antenna element; and
- an interrogatable system comprising a second transmission and reception antenna element;
- said device comprising an expandable tubular structure (42), said structure integrating a biocompatible material (46) and said acoustic wave sensor (24) being encapsulated in said biocompatible material (46);
- said second antenna element operating at frequencies above several tens of megahertz,
**characterised in that** said sensor is a pressure and temperature sensor comprising a first reference resonator (38), a second temperature-measuring resonator (40) and a third pressure-sensitive resonator (36).

2. The remotely interrogatable pressure and temperature measuring device according to claim 1, **characterised in that** said tubular structure comprises a metal structure made of expandable mesh.

3. The remotely interrogatable pressure and temperature measuring device according to claim 1 or 2, **characterised in that** said sensor is located on an internal wall of said tubular structure.

4. The remotely interrogatable pressure and temperature measuring device according to any one of claims 1 to 3, **characterised in that** said biocompatible material is a resin.

5. The remotely interrogatable pressure and temperature measuring device according to any one of claims 1 to 4, **characterised in that** said tubular structure comprises a non-expandable rigid part, in which said sensor is integrated.

6. The remotely interrogatable pressure and temperature measuring device according to any one of claims 1 to 5, **characterised in that** said sensor is a pressure and temperature sensor comprising a stack of a plurality of substrates each comprising a resonator and an integrated antenna, produced by means of peripheral walls.

7. The remotely interrogatable pressure and temperature measuring device according to claim 6, **characterised in that** said peripheral walls are of the glass mosaic type (pv).

8. The remotely interrogatable pressure and temperature measuring device according to any one of claims 6 or 7, **characterised in that** said pressure-sensitive resonator is in an upper position in the stack and in a head-to-tail position relative to said reference resonator.

9. The remotely interrogatable pressure and temperature measuring device according to any one of the preceding claims, **characterised in that** said system operates in the ISM band at 434 MHz.

10. A method for manufacturing a remotely interrogatable pressure and temperature measuring device according to any one of claims 1 to 9, **characterised in that** it comprises the following steps:
- producing an opening on a peripheral part of an expandable tubular structure;
- placing said expandable tubular structure on a mandrel having a groove;
- positioning said sensor in said opening of said tubular structure positioned on said mandrel and facing said groove, said sensor comprising a first reference resonator, a second temperature-measuring resonator and a third pressure-sensitive resonator;
- depositing a biocompatible coating resin allowing said sensor to be encapsulated;
- removing said tubular structure from said mandrel.
